# EUROPEAN PATENT SPECIFICATION

(11) **EP 4 216 814 B1**
(45) Date of publication and mention of the grant of the patent: **05.03.2025**
(21) Application number: 21782694.0
(22) Date of filing: 21.09.2021
(51) Int. Cl.: A61B 5/11, A61B 5/00, A61B 5/113, A61B 5/024

(54) **METHOD AND SYSTEM FOR NON-CONTACT VITAL SIGN MONITORING**
VERFAHREN UND SYSTEM ZUR KONTAKTLOSEN VITALPARAMETERÜBERWACHUNG
PROCÉDÉ ET SYSTÈME DE SURVEILLANCE DE SIGNES VITAUX SANS CONTACT

(30) Priority: 23.09.2020 US 202063082197 P
(43) Date of publication of application: 02.08.2023
(73) Proprietor: Analog Devices International Unlimited Company, Limerick (IE)
(72) Inventor: KAEMMERER, Christoph, 81476 München (DE); TRAA, Johannes, Medford, Massachusetts 02155 (US)
(74) Representative: Horler, Philip John
(86) International application number: PCT/EP2021/075973
(87) International publication number: WO 2022/063792

(56) References cited:
- US-A1- 2007 118 054
- US-A1- 2017 042 471

## Description

### TECHNICAL FIELD

The present disclosure generally relates to hardware configurations and signal processing methods for tracking heart and/or respiratory rates in accordance with some embodiments.

### BACKGROUND

Humans exhibit four major vital signs that are used to provide a comprehensive picture of an individual's bodily vital functions. These four vital signs (body temperature, heart rate, breath rate, and blood pressure) of an individual allow caregivers to assess whether further measures are to be taken with regards to an individual's overall health. Being able to obtain any of the four vital signs without being tethered to monitoring equipment invites novel and useful ways for humans to keep track of their health. However, monitoring vitals without having the subject in physical contact with monitoring equipment may be difficult, costly, or may produce unreliable data.

US-A1-2007/118054 describes methods and systems for monitoring vital signs for the prediction and treatment of physiological ailments.

US-A1-2017/042471 describes a method and system for detecting sleep phenomena including sleep cycles and sleep disorders such as sleep apnea and hypopnea.

### SUMMARY

A novel approach to heart and respiratory rate monitoring is achieved by utilizing ballistocardiographs. Ballistocardiographs are graphical representations of the movement of a person's body based on an upward recoil caused by the movement of blood through the heart. This low frequency vital sign can be measured using noninvasive methods.

However, measuring and recording this vital sign (e.g., heart rate) of a person is difficult using noninvasive methods as the signal is easily overcome by noise and external vibration. To maintain signal integrity, typically, a user or a person whose vital signals are being monitored is in direct contact with measurement equipment and for the user to be as still as possible. The technical problem of obtaining a user's vital sign without requiring the user to remain still and be in direct contact with equipment begs to be solved.

Accordingly, the inventors have recognized, among other things, a need for a contact-free, noninvasive technique for monitoring vital signs of a user and have discovered a technical solution to this technical problem. Non-contact vital signal monitoring using microelectromechanical (MEMS) accelerometers allows measurements of heart and respiratory rates to be made without requiring direct contact with the user or person being monitored. The signals measured by one or more low noise, MEMS accelerometers are filtered to detect and monitor the heart and or respiratory signal of the user while the person being monitored is sitting or even lying down in a chair or in a bed. In addition to obtaining measurements of heart and respiratory rates, the embodiments of the disclosure herein contemplate capturing additional heart and lung related metrics including, but not limited to snoring and sleep apnea events. The invention provides a method according to claim 1, a signal acquisition system according to claim 7 and a non-transitory computer readable storage medium according to claim 13. The scope of the invention is defined by the appended claims For better understanding of the invention the present disclose provides also further examples, which do not fall under the scope of the claims.

In some embodiments, a method includes receiving a first signal from a first accelerometer channel, the first signal including information indicative of a physiologic signal obtained extra-corporeally. The method further includes receiving a second signal from a second accelerometer channel. The second signal includes information indicative of the physiologic signal obtained extra-corporeally. The method further includes identifying a base frequency of the physiologic signal using at least one harmonic template from a plurality of harmonic templates from at least one of the first and second signals. For at least one of the first and second signals or an aggregated representation thereof, apply a filter to establish a tracking signal. the tracking signal is initialized with the identified base frequency. The method further includes storing and/or transmitting the tracking signal as a representation of the physiologic signal.

In some embodiments, a signal acquisition system configured to track a physiologic signal includes a first accelerometer having a first accelerometer channel configured to obtain information of a physiologic signal obtained extra-corporeally and a second accelerometer having a second accelerometer channel distinct from the first accelerometer channel configured to obtain information of the physiologic signal obtained extra-corporeally. The signal acquisition system also includes a control circuit configured to receive a first data signal from the first accelerometer channel, receive a second data signal from the second accelerometer channel, and identify a base frequency of the physiologic signal from at least one of the first and second signals. The harmonic template is one of a plurality of harmonic templates. The control circuit of the signal acquisition system also applies a filter to at least one of the first and second signals or an aggregated representation thereof, to establish a tracking signal. The tracking signal is initialized with the identified base frequency. The control circuit of the signal acquisition system is further configured to store or transmit the tracking signal as a representation of the physiologic signal.

Method examples described herein can be machine or computerimplemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

### BRIEF DESCRIPTION OF THE DRAWINGS

To easily identify the discussion of any element or act, the most significant digit or digits in a reference number refer to the figure number in which that element is first introduced.
FIG. 1 illustrates a diagram of an example environment including a vital signal monitoring system in accordance with some embodiments.
FIG. 2 illustrates a block diagram of the system including heart rate, heart rate variability, and respiration rate pipelines in accordance with some embodiments.
FIG. 3 is a flow chart illustrating operations of example methods for providing non-contact vital signal monitoring in accordance with some embodiments.
FIG. 4 is a flow chart illustrating operations of an alternative example method for providing non-contact vital signal monitoring in accordance with some embodiments.
FIG. 5 is a representation of an example comprising a heart rate and respiration rate signals of the system described herein, according to some embodiments.
FIG. 6 is a block diagram illustrating components of a device able to read instructions from a machine readable medium (e.g., machine-readable storage medium or machine-readable storage device) and perform any one or more of the methodologies discussed herein.

### DETAILED DESCRIPTION

The present inventors have recognized, among other things, that monitoring of physiologic signals can present various challenges. For example, in one approach, such monitoring can require a subject to be physically tethered (via cables or body-worn sensors) to a machine. The user may also have to remain relatively still, or the acquisition of such signals may be corrupted.

The present subject matter concerns, among other things, apparatus, and techniques for monitoring physiologic signals such as heart rate or respiratory rates. Such monitoring is performed without requiring a subject being monitored to be physically tethered to a machine.

Accordingly, the present disclosure describes a method and systems for non-contact vital sign monitoring that allows detection, recordation, and/or transmission of human vital signs without necessitating physical contact with measurement equipment.

Reference will now be made to embodiments, examples of which are illustrated in the accompanying drawings. In the following description, numerous specific details are set forth to provide an understanding of the various described embodiments. However, it will be apparent to one of ordinary skill in the art that the various described embodiments may be practiced without these specific details. In other instances, well-known methods, procedures, components, circuits, and networks have not been described in detail so as not to unnecessarily obscure aspects of the embodiments.

It will also be understood that, although the terms first, second, etc. are, in some instances, used herein to describe various elements, these elements should not be limited by these terms. These terms are used only to distinguish one element from another. For example, a first accelerometer could be termed a second accelerometer, and, similarly, a second accelerometer could be termed a first accelerometer, without departing from the scope of the various described embodiments. The first accelerometer and the second accelerometer are both accelerometers, but they are not necessarily the same accelerometer.

The terminology used in the description of the various described embodiments herein is for the purpose of describing embodiments only and is not intended to be limiting. As used in the description of the various described embodiments and the appended aspects, the singular forms "a," "an," and "the" are intended to include the plural forms as well, unless the context clearly indicates otherwise. It will also be understood that the term "and/or" as used herein refers to and encompasses all possible combinations of one or more of the associated listed items. It will be further understood that the terms "includes," "including," comprises," and/or "comprising," when used in this specification, specify the presence of stated features, integers, steps, operations, elements, and/or components, but do not preclude the presence or addition of one or more other features, integers, steps, operations, elements, components, and/or groups thereof. The term "exemplary" is used herein in the sense of "serving as an example, instance, or illustration" and not in the sense of "representing the best of its kind."

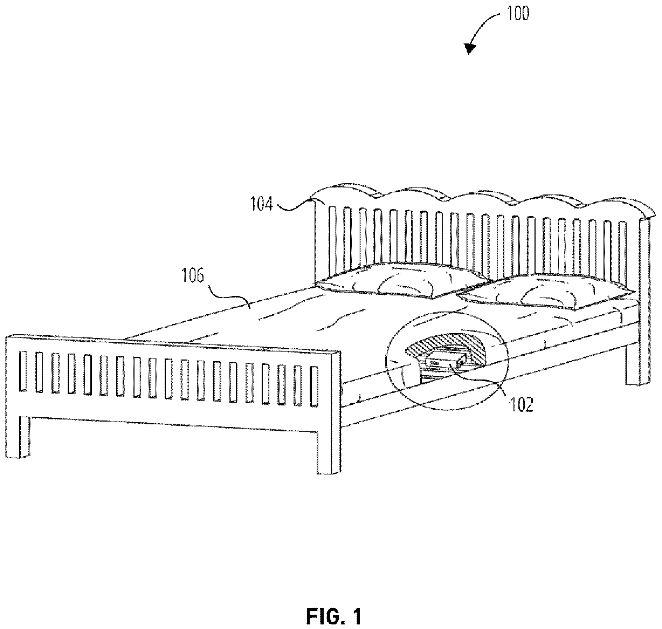

FIG. 1 illustrates a diagram of an example environment including a vital signal monitoring system in accordance with some embodiments.

System 100 includes a bed frame 104, a mattress 106, and a non-contact vital signal monitoring system 102. As described in more detail herein, the system 100 includes a setup for a patient (e.g., user) to recline or rest in a supine, prone, right/left lateral recumbent, seated, or other various positions on the mattress 106. In some embodiments, the non-contact vital signal monitoring system 102 is embedded within the mattress 106. In some other embodiments, the non-contact vital signal monitoring system 102 is positioned proximate the mattress (e.g., on or within the bed frame 104, not shown).

In some embodiments, one or more accelerometers are configured at a specified location on a bed. The observed configuration is for two accelerometers on top of the mattress, close to a location where a user would lie and another accelerometer on the ground. The accelerometer on the ground is still within a reasonable range to detect vital signs from the person on the bed. In some embodiments, additional sensors and accelerometers may be placed proximate the mattress in various configurations. For example, an additional accelerometer may be placed beside the patient within a first range (e.g., 6 inches away from the user) primarily for noise filtration. The accelerometer data from the one or more accelerometers can be used to filter out the outside vibrations (e.g., noise) by aggregating the data sets from the one or more accelerometers. After the data signals are process through one or more filters, the physiologic signal can be recovered by using known harmonic templates of the physiologic signal. For example, a known heart rate for a user/patient is within a range of 0 to 5 Hz and has a specific wave form. This known heart rate is used as a harmonic template of the tracked physiologic signal to differentiate the tracked (e.g., physiologic) signal from the received signal.

In some embodiments, the configuration of the one or more accelerometers includes mounting the one or more accelerometers to a rigid plate to reduce noise and vibrations. One or more additional accelerometers are used to filter out additional external noise (e.g., walking, clapping, shouting sounds). The one or more additional accelerometers are useful to enhance the signal quality and to improve fidelity of the tracked signal.

The system may include one or more low noise accelerometers (e.g., ADXL355 available from Analog Devices, Inc., Wilmington, MA, USA, or another accelerometer). The system may also include one or more electronic devices having a processor configured to receive, process, transmit, and/or analyze data from the one or more low noise accelerometers. The one or more accelerometers may be configured on a rigid structure and placed in distinct locations with respect to the other one or more accelerometers. The mechanical movements of the heart can be detected by one or more accelerometers placed within a reasonable distance of a patient or user of the device without being in direct contact with the device. Each of the one or more accelerometers are coupled to a processor to ensure the raw signals are timestamped and recorded. In this manner, the accelerometer can operate as a ballistocardiograph to measure impact and the acceleration, such as caused by the mechanical movements of the heart.

The non-contact vital signal monitoring system may include one or more accelerometers (e.g., 1^{st}, 2^{nd}, 3^{rd} eval boards) in communication with and/or coupled to a microcontroller (e.g., STM32 Nucleo-144) via insulation-displacement contact (IDC) connectors.

In some embodiments, the vital signal monitoring system may be coupled to various external devices for further analysis and/or distribution. In such embodiments, the non-contact vital signal monitoring system may include one or more cables coupling the monitoring system to the various external devices.

Additional considerations include introducing a rigid plastic plate beneath one or more of the accelerometers to house the accelerometer, signal processing and data transmission device to increase the sensitivity. This can be achieved by physically affixing the housing to the frame of a chair and/or a bed.

Additionally, or alternatively, one or more additional accelerometers may be added to the system or used in tandem with the current system to provide further noise filtration.

Additionally, or alternatively, various other vital signals can be obtained through this method and system including blood pressure, body temperature, and others.

Additionally, or alternatively, the system can be further configured to provide early warning scores and sleep quality indicators based on the tracked heart rate and/or respiration rate.

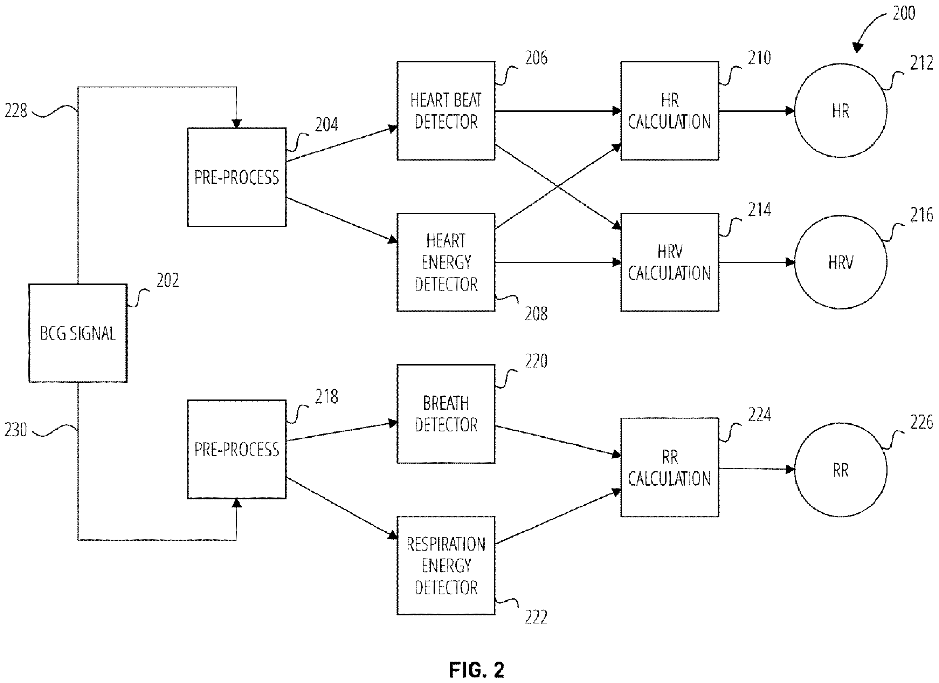

FIG. 2 illustrates a block diagram of the system including heart rate and heart rate variability pipeline 228, and respiration rate pipeline 230 in accordance with some embodiments.

Block diagram 200 shows an overview of the system for implementing non-contact vital signal monitoring to determine a heart rate, a heart rate variability, and a respiration rate using a frequency-domain heart rate estimator. The block diagram 200 illustrates how a received ballistocardiogram (BCG) signal 202 is processed to provide a heart rate 212, a heart rate variability 216, and a respiration rate 226 in accordance with some embodiments.

For each of the heart rate 212, heart rate variability 216, and respiration rate 226 estimators, a one or more pre-processing operations 204 and/or 218 including detrending operations are performed on the raw input signal to remove the effects of gravity and slow variation from the ballistocardiogram signal 202. In some embodiments, the pre-processing operations 204 and pre-processing modules 218 including detrending operations are performed independently for the heart rate and heart rate variability estimation and a separate operation is performed for the respiration rate estimation. In some embodiments, for each of the estimators, a corresponding energy detection module monitors user's status and is configured to determine if the signal becomes unusable. In the instances the signal of the estimators becomes unstable, the trackers reset, and the output of the estimators become undefined for some time while the trackers reset.

To process the received signal for each estimator, the signal is (i) filtered through a bandpass filter, (ii) processed through a heartbeat time estimator, and (iii) processed through a heart rate and/or heart rate variability calculator. First, the signal is filtered through a bandpass filter (e.g., a fourtap Butterworth bandpass filter) between a specified frequency (e.g., 2-10 Hertz). The bandpass filter is applied on the signal in the forwards and backwards direction. The output of the filter is a signal with a squared magnitude response also having a non-linear phase response having been zeroed out.

Second, the filtered signal is used to estimate the heartbeat times. To do so, a peak-trough pattern is identified in the time domain. In some embodiments, a signal sample is determined to be at a peak if the signal is larger or equal to its immediate neighbors. The determined peak value is validated if the peak value is large in comparison to neighboring values within at least two, time windows. Of the two-time windows, one is a smaller window and covers a duration less than an average heartbeat. The smaller window serves to identify the main peak. A second window is a larger window and covers a duration of multiple heart beats. This larger second window serves to filter out spurious peaks of signal activity.

The beat times are identified as being validated signal points with a peak-trough or trough-peak pattern occurring in quick succession (e.g., within 1 second). In some embodiments either of the peak-to-trough or trough-to-peak patterns are determined as being the heartbeat time. The successive difference between the heartbeat time is calculated as the beat-to-beat interval. In some embodiments, to filter out outliers, a local median of beat-to-beat intervals is computed, smoothed, and any intervals lying outside of a predetermined range (e.g., +/- 5%) is rejected.

Using the calculated beat-to-beat intervals, a heart rate is estimated as the reciprocal of the median beat-to-beat time interval within a time window (e.g., the most recently recorded 5 seconds of the signal). The heart rate variability is estimated as being the average absolute difference from the median in a desired time window (e.g., the most recently recorded 30 seconds). In some embodiments, a constant is added to the heart rate variability estimate to compensate for the downward bias of using the median value. Additionally, the median value can in some embodiments be discontinuous. To assuage the discontinuity, a 1-tap, exponential decay infinite impulse response (IIR) filter is used to smooth the discontinuity.

Additional operations can be performed on the signal including blind equalization. The harmonic stack of the heart signal exhibits varying resonances depending on the user's position, orientation, body type, mattress characteristics, bed frame characteristics, and others. To generalize these varying conditions, a learned finite impulse response (FIR) filter is applied to flatten out the spectral envelope of the harmonic stack. In some embodiments, a least-squares optimization of a linear phase FIR filter is applied on a segment of the signal (e.g., 3 seconds). This process of blind equalization sharpens the heartbeat peaks of the signal in the time-domain. Further, in some embodiments, axis combination is performed to obtain valuable data from the received signal. The X-axis of the hart signal is seen to have a peak in a positive direction when the accelerometer's x-axis is oriented toward the foot of the bed. Combining the signal of the x-axis with the signal of the y-axis and/or the z-axis, yields further information including in some embodiments, a suggestion of a lateral ballistic force being exerted by the user's heart. Additional information includes a possibility of a physical setup introducing delay into the signal that is axisdependent and is to be accounted for.

The respiration signal processing pipeline is run at 5 Hz or lower. Typically, the respiration signal energy is below 0.5 Hz and is sinusoidal. As described above with respect to detrending the signal, a lowpass filter is applied (e.g., 4-tap lowpass Butterworth filter) to remove any frequencies above 0.5 Hz. Subsequently, the peaks and troughs of the signal are identified (similar to the operations in heartbeat detection). Further, a peak-to-peak and trough-to-trough time is calculated for the respiration signal and merged together. From the time intervals of the peak-to-peak and trough-to-trough, a respiration rate can be estimated as the reciprocal of the media time interval in a ageionated time window. The median time interval can be discontinuous and, in some embodiments, smoothed using a 1 tap exponential decay IIR filter.

The methods and systems described herein are capable of tuning and being tuned to obtain high-quality signals and subject-to-sensor coupling. Various parameters for tuning include adjustable minimum and maximum signal power thresholds, and minimum and maximum heart rate, heart rate variability, and respiration rate.

In an example, a user (e.g., patient) is situated on a mattress with an embedded sensing mechanism (e.g., non-contact vital signal monitoring system 102). There are three estimators operating within the embedded sensing mechanism while the user is situated on a mattress. A first energy detector for a heart rate pipeline (e.g., heart rate 212) recursively processes the received signal, sample-by-sample. The heart rate and heart rate variability estimations are conducted at a low sampling rate (e.g., 200 Hz). The heartbeat detector 206 may perform peak detection, peak validation, and the HR calculation 210 module may, using the output of the heartbeat detector 206, perform identification of peak-trough pairs, beat-to-beat internal validation, and compute the heartbeat rate (e.g., in beats per minute). The heart energy detector 208 may in some embodiments, share similar functionalities with the heartbeat detector 206. heart energy detector 208 may also perform functionalities including beat-to-beat validation. The output of the heart energy detector 208 is submitted to HRV calculation 214 that performs a calculation to determine the heart rate variability of the obtained signal. In some embodiments, the HRV calculation 214 module receives one or more signals, data samples, or other information from either or both heartbeat detector 206 and heart energy detector 208 modules.

A second energy detector for a respiration rate pipeline (e.g., respiration rate 226) similarly to the heart rate pipeline, recursively processes the received signal, sample-by-sample. The respiration rate pipeline includes signal pre-process module 218 which may include down sampling by applying a lowpass filter and subsampling from a higher frequency to a lower frequency (e.g., from 200 Hz down to 5 Hz). Additional pre-process module 218 operations include applying filters as needed. The breath detector 220 module may perform operations including identifying peaks and troughs. The respiration energy detector 222 module may perform operations including calculating respiration signal energy and thereby determining what portions of the signal are usable for estimating respiration rate. The respiration rate (RR) calculation block (e.g., RR calculation 224) may be used to take a median respiration rate value as the respiration rate 226 output. The RR calculation 224 may output the median respiration rate value at a specified time interval (e.g., once per second).

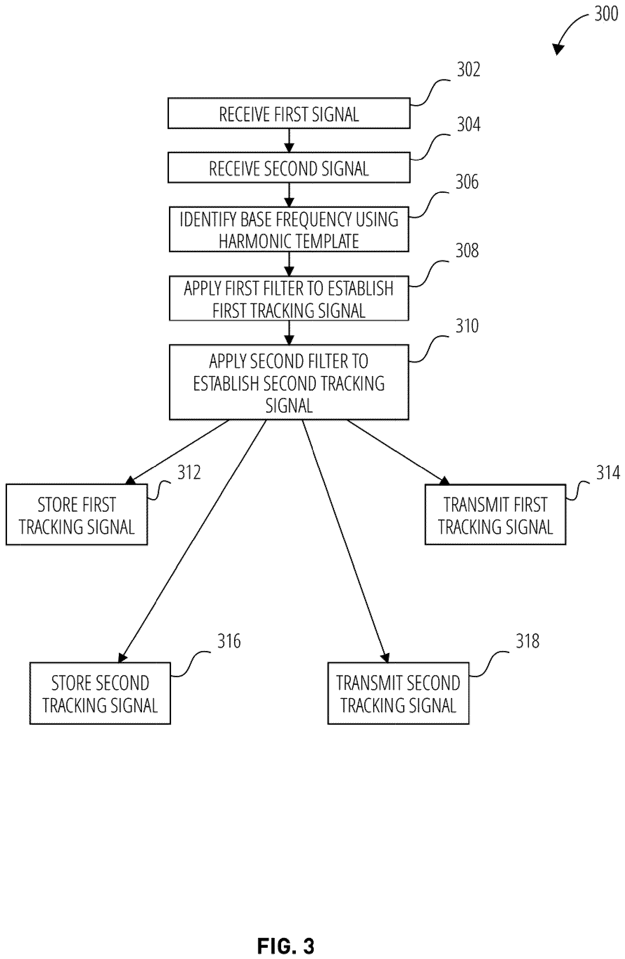

FIG. 3 is a flow chart illustrating operations of example methods for providing non-contact vital signal monitoring in accordance with some embodiments. It will be understood that the method 300 may be performed by a device, such as a computing device executing instructions of a software system. For instance, operations of a method 300 may be represented by executable instructions (e.g., software) that, when executed by a processor of a computing device, cause the computing device to perform the method 300. Thus, an operation of the method 300 may be performed by a hardware processor (e.g., central processing unit) of a computing device (e.g., desktop computer, server, etc.). Accordingly, the method 300 is described below in reference to such a computing device. Depending on the embodiment, an operation of the method 300 may be repeated in different ways or involve intervening operations not shown. Though the operations of the method 300 may be depicted and described in a certain order, the order in which the operations are performed may vary among embodiments, including performing certain operations in parallel.

Method 300 begins at operation 302 where a computing device receives a first signal. The first signal is obtained from a first accelerometer channel, extra-corporeally (e.g., without making physical contact with a user).

At operation 304, the computing device receives a second signal. The second signal may be received from a second accelerometer channel, the second signal is also obtained extra-corporeally. The first signal can be obtained by a first accelerometer channel (e.g., measuring a y axis) and the second signal can be obtained by a second accelerometer channel (e.g., measuring an x axis).

In some embodiments, both the first and the second accelerometer channels are channels of a single accelerometer unit. In some embodiments, the single accelerometer unit may have more channels. In some other embodiments, the first and second accelerometer channels may be from separate or distinct accelerometer units. In some embodiments, the computing device receives a third signal measuring the physiologic signal from a third accelerometer channel (e.g., measuring a z axis) where the third accelerometer is distinct from the first and second accelerometer channels. In some embodiments, the first accelerometer channel, the second accelerometer channel, and/or the third accelerometer channels are channels of a solid-state accelerometer. In some embodiments, the first, second, and/or third accelerometer channels define orthogonal sensing axes. For example, the first channel measures a y-axis, the second channel measures an x-axis, and third channel measures a z-axis.

The computing device at operation 306 identifies a base frequency using a harmonic template. The computing device may use at least one harmonic template from amongst a plurality of harmonic templates to identify a base frequency of a physiologic signal (e.g., heart rate, respiration rate) from at least one of the first and second signals. In some embodiments, operations 308-310 are performed for at least one of the first and second signals or an aggregated representation of the first and second signals.

In some embodiments, the identified base frequency is constrained to a specified tracking frequency range (e.g., 0-10 Hz).

In response, the computing device applies a first filter to establish a first tracking signal at operation 308. Applying the first filter can include applying a bandpass filter on at least one of the first and second signals or an aggregated representation thereof to generate a first intermediate signal. The first intermediate signal is then reversed, and the bandpass filter is applied on the reversed first intermediate signal to establish the first tracking signal.

In some embodiments, after applying the first filter to establish the tracking signal, the computing device detects that the tracking signal cannot be obtained and sends a notification to an electronic device that the first tracking signal is lost. Detection of the first tracking signal includes monitoring whether a central tendency of noise level values is higher than a current noise level by a specified amount, and in accordance with a determination that the central tendency of noise level values is higher than the current noise level by the specified amount and for a specified duration, declaring the status of the tracking signal as being unavailable. In some embodiments the status of the tracking signal as being unavailable is sent to an electronic device. The tracking signal can be lost or unattainable for various reasons including low signal integrity, high noise, disrupted transmission of the signal, and many more. In some embodiments, the cause of the lost or unattainability of the tracking signal is transmitted to the electronic device.

A second filter is then applied by the computing device at operation 310 to establish a second tracking signal. Applying a second filter to establish a second tracking signal can include applying a lowpass filter to generate a second intermediate signal and applying a smoothing filter on the second intermediate signal to establish the second tracking signal.

In some embodiments, the computing device stores the first tracking signal (operation 312) and also stores the second tracking signal (operation 316).

In some embodiments, the first tracking signal is a representation of a heart rate signal. In some embodiments, the first tracking signal represents a heart rate variability signal.

In some embodiments, the second tracking signal represents a respiration rate signal.

In some embodiments, the computing device transmits the first tracking signal (operation 314) and/or transmits the second tracking signal operation 318).

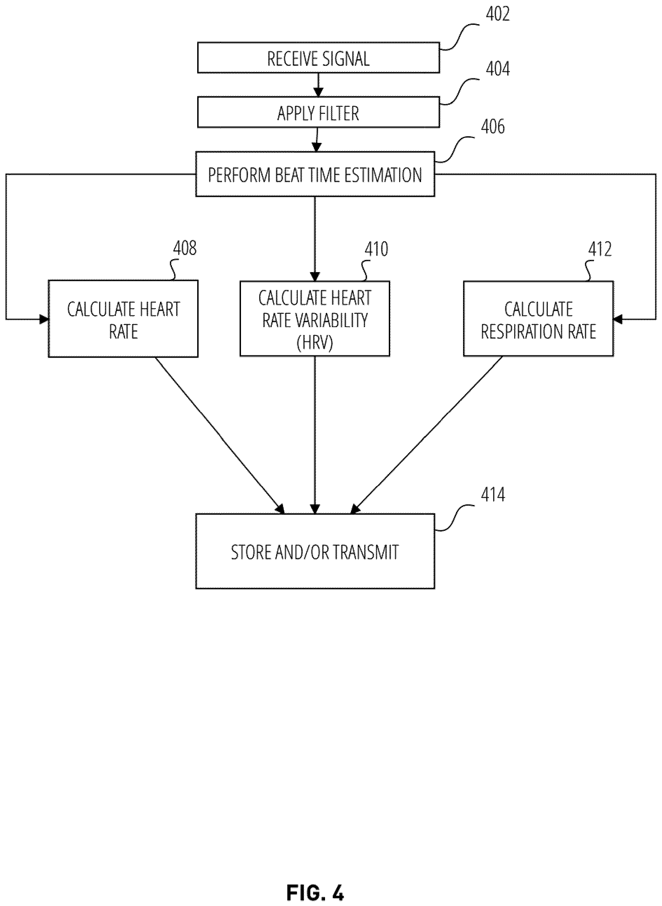

FIG. 4 is a flow chart illustrating operations of an alternative example method for providing non-contact vital signal monitoring in accordance with some embodiments.

It will be understood that the method 400 may be performed by a device, such as a computing device executing instructions of a software system. For instance, operations of a method 400 may be represented by executable instructions (e.g., software) that, when executed by a processor of a computing device, cause the computing device to perform the method 400. Thus, an operation of the method 400 may be performed by a hardware processor (e.g., central processing unit) of a computing device (e.g., desktop computer, server, etc.). Accordingly, the method 300 is described below in reference to such a computing device. Depending on the embodiment, an operation of the method 400 may be repeated in different ways or involve intervening operations not shown. Though the operations of the method 400 may be depicted and described in a certain order, the order in which the operations are performed may vary among embodiments, including performing certain operations in parallel.

Method 400 begins at operation 402 where a computing device receives a signal. The signal is obtained from an accelerometer channel, extra-corporeally (e.g., without making physical contact with a user) at a first time window. In some embodiments, the physiologic signal is continuously received by the computing device (e.g., an ongoing signal read). At operation 404, the computing device applies a filter on the physiologic signal to obtain a filtered physiologic signal. In some embodiments, the filter is a bandpass filter. Additionally and/or alternatively, the computing device may apply an equalization operation on the filtered physiologic signal.

At operation 406, the computing device performs a beat time estimation on the filtered physiologic signal within the first time window (e.g., a five second window). In computing the beat time estimation, the computing device may determine peak samples of the filtered physiologic signal and trough samples of the filtered physiologic signal. The computing device then calculates beat times as a function of the peak to trough samples and calculates a median of beat times as being the beat time estimation. Based on the beat time estimation, the computing device can calculate a heart rate (operation 408), a heart rate variability (410), and a respiration rate (412). In some embodiments, any of the heart rate, HRV, and respiration rates can be calculated for a specified time segment (e.g., for a 5 second time window).

In some embodiments, in calculating the respiration rate (operation 412), the computing device applies a detrending operation to generate a detrended physiologic signal. The computing device then applies a second filter on the detrended physiologic signal to generate a filtered detrended physiologic signal. The second filter (e.g., a low-pass filter) may be distinct from the filter of operation 404. The computing device then calculates peak-to-peak times of the filtered detrended physiologic signal and calculates the respiration rate. In some embodiments, the computing device also calculates trough-to-trough times of the filtered detrended physiologic signal and uses both the peak-to-peak and trough-to-trough times to calculate the respiration rate.

At operation 414, the computing device stores and or transmits the heart rate, heart rate variability, respiration rate, and/or the filtered physiologic signal.

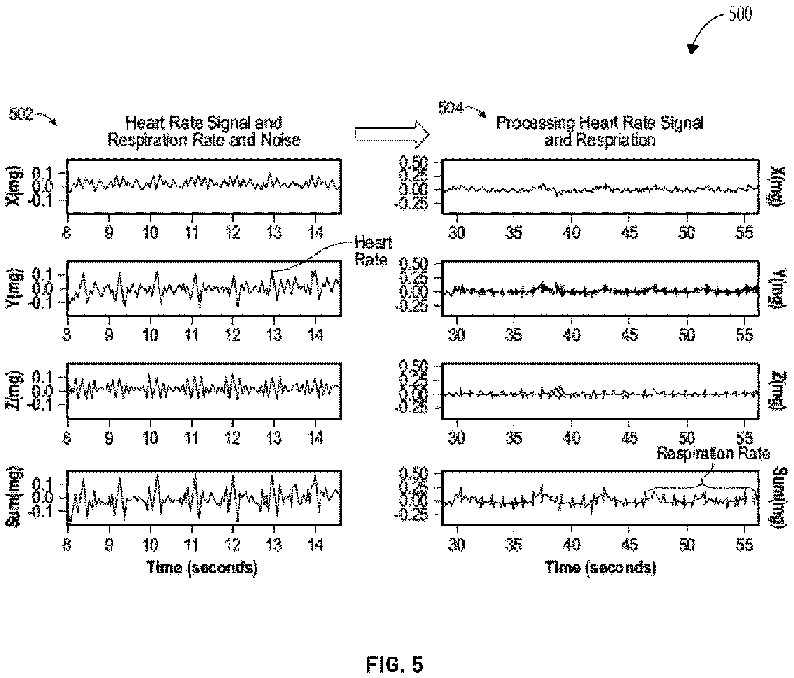

FIG. 5 is a representation of an example signal graph 500 comprising a heart rate and respiration rate signals of the system described herein, according to some embodiments.

An algorithm for determining a tracker signal (e.g., heart rate or respiration rate) includes locking onto and tracking harmonic stacks in the ballistocardiograph's short-time spectrum. In one embodiment, a bank of trackers is instantiated, each tuned to an integer multiple of an estimated fundamental frequency of the tracked signal (e.g., heart rate, respiration rate). Each of the trackers will also have a bandpass filter tuned to the same integer multiple of the estimated fundamental frequency to isolate one harmonic. In another embodiment, a sparse multiplication function is applied to the harmonic stack templates of the trackers to compute the tracking signal around an estimated fundamental frequency at each frame of the ballistocardiograph.

The received data signals from the accelerometers 502 are filtered to process out vibration and noise that fall out of the frequency range of the heartbeat and/or respiration rate (e.g., 0 to 5 Hz). Noise outside of the frequency range is most likely external noise from the user/ patient or environmental noise.

In some embodiments, more than one harmonic of the tracked physiologic signal is used to derive the fundamental frequency (F0) to obtain the tracked physiologic signal representing a user's underlying physiologic activity.

In some embodiments, the received signals are processed using various operations described herein. For example, for overlapping blocks of samples (e.g., 1000 samples with 4/5 temporal overlap @ 200 Hz sampling rate), a spectrum of the block is determined. The computed magnitude may be computed with a fast Fourier transform (FFT). Second, an inner product of the magnitude spectrum is calculated with each of a sparse harmonic comb template to obtain the energy values at each fundamental frequency (F0). Third, update the tracker with the comb energies and fourth, report the current fundamental frequency (F0) estimate and the tracker's status.

In some embodiments, the tracker is established by using alpha-beta filters. Establishing the tracker signal with alpha-beta filters include: performing a prediction using the alpha-beta filter, detecting the tracker by finding the peak near the tracker's center and evaluating the peak quality score (PQS) discussed in further detail below, and updating the status of the tracker if the PQS score is consistently high, with a "tracking" status, if the score is not consistently high, with a "lost" status, and further re-initializing the tracking with a "tentative" status.

In some embodiments, octave errors may be detected in the tracker that may require correcting. In some embodiments, there are occasions when there are not enough harmonics in the frequency-domain signal. To address this, in some embodiments, the tracker is tracked at half of the fundamental frequency (F0) and a peak quality score (PQS) is evaluated. If the calculated PQS for the tracker is higher than a threshold value, the tracked F0 is tracked to the peak of the F0.

In accordance with some embodiments, re-initializing tracking may occur at the beginning of a session, after a noise burst is detected, or when the tracker signal is lost for a predetermined period. Re-initializing the tracking includes initializing multiple alpha-beta filter instances on the largest peaks in the current frame and tentatively tracking each of them for a predetermined number of frames. If one or more of the tentative tracks has a PQS signal that meets a threshold requirement, the track with the highest PQS score is selected and tracked.

In some embodiments, a peak quality score is calculated when tracking the physiologic signal. As in some embodiments, the tracking signal gets lost and is then re-initialized to begin tracking the physiologic signal again. To do so, a peak quality score is calculated. The peak quality score is calculated by evaluating the prominence of the peak closes to the tracked signal. This can be implemented by dividing the peak value by the average of the median values in the windows immediately to the left and to the right of the peak.

As shown, the processed heart rate signal and respiration rate signals 504 are devoid of noise and extraneous information. The processed respiration rate signals 504 may be sent to an external electronic device (e.g., a physician's computer) for monitoring and/or further analysis.

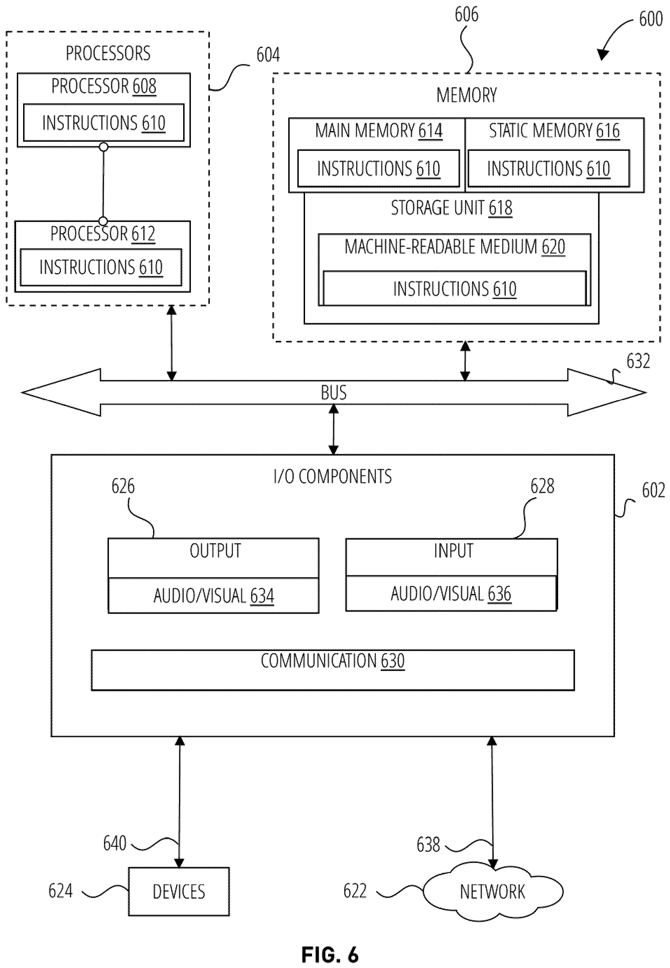

FIG. 6 is a block diagram illustrating components of a device 600 according to some embodiments, able to read instructions from a machine readable medium (e.g., machine-readable storage medium or machine-readable storage device) and perform any one or more of the methodologies discussed herein. The instructions may cause the device 600 to execute the method illustrated in FIG. 3 and/or FIG. 4. Additionally, or alternatively, the instructions 610 may implement one or more of the components of FIGs. 1-5. The instructions 610 transform the general, non-programmed device 600 into a particular device 600 programmed to carry out the described and illustrated functions in the manner described. In alternative embodiments, the device 600 operates as a standalone device or may be coupled (e.g., connected) to other machines.

The device 600 may comprise, but not be limited to, a server computer, a client computer, a personal computer (PC), a tablet computer, a laptop computer, a netbook, a PDA, or any machine capable of executing the instructions 610, sequentially or otherwise, that specify actions to be taken by device 600. Further, while only a single device 600 is illustrated, the term "machine" shall also be taken to include a collection of devices that individually or jointly execute the instructions 610 to perform any one or more of the methodologies discussed herein.

The device 600 may include processors 608, memory/storage 606, and I/O components 602, which may be configured to communicate with each other such as via a bus 632. In an example embodiment, the processors 604 (e.g., a Central Processing Unit (CPU), a Reduced Instruction Set Computing (RISC) processor, a Complex Instruction Set Computing (CISC) processor, a Graphics Processing Unit (GPU), a Digital Signal Processor (DSP), an ASIC, a Radio-Frequency Integrated Circuit (RFIC), another processor, or any suitable combination thereof) may include, for example, processor 608 and processor 612 that may execute the instructions 610. The term "processor" is intended to include multi-core processor that may comprise two or more independent processors (sometimes referred to as "cores") that may execute instructions 610 contemporaneously. Although FIG. 6 shows multiple processors 604, the device 600 may include a single processor with a single core, a single processor with multiple cores (e.g., a multi-core process), multiple processors with a single core, multiple processors with multiples cores, or any combination thereof.

The memory/storage memory 606 may include a memory 606, such as a main memory 614, static memory 616, or other memory storage, and a storage unit 618, both accessible to the processor 608 such as via the bus 632. The storage unit 618 and memory 620 store the instructions 610 embodying any one or more of the methodologies or functions described herein. The instructions 610 may also reside, completely or partially, within the memory 606, within the storage unit 618, within at least one of the processors 608 (e.g., within the processor's cache memory), or any suitable combination thereof, during execution thereof by the device 600. Accordingly, the memory 614, the storage unit 618, and the memory of processors 608 are examples of machine-readable media.

As used herein, "machine-readable medium" includes a machine-readable storage device able to store instructions 610 and data temporarily or permanently and may include, but is not limited to, random-access memory (RAM), read-only memory (ROM), buffer memory, flash memory, optical media, magnetic media, cache memory, other types of storage (e.g., Erasable Programmable Read-Only Memory (EEPROM)) and/or any suitable combination thereof. The term "machine-readable medium" should be taken to include a single medium or multiple media (e.g., a centralized or distributed database, or associated caches and servers) able to store instructions 610. The term "machine-readable medium" shall also be taken to include any medium, or combination of multiple media, that is capable of storing instructions (e.g., instructions 610) for execution by a device (e.g., device 600), such that the instructions, when executed by one or more processors of the device 600 (e.g., processors 604), cause the device 600 to perform any one or more of the methodologies described herein. Accordingly, a "machine-readable medium" refers to a single storage apparatus or device, as well as "cloud-based" storage systems or storage networks that include multiple storage apparatus or devices. The term "machine-readable medium" excludes signals per se.

The input/output (I/O) components 602 may include a wide variety of components to receive input, provide output, produce output, transmit information, exchange information, capture measurements, and so on. The specific I/O components 602 that are included in a particular machine will depend on the type of machine. For example, portable machines such as mobile phones will likely include a touch input device or other such input mechanisms, while a headless server machine will likely not include such a touch input device. It will be appreciated that the I/O components 602 may include many other components that are not shown in FIG. 6. The I/O components 602 are grouped according to functionality merely for simplifying the following discussion and the grouping is in no way limiting. In various example embodiments, the I/O components 602 may include output components 626 and input components 628. The output components 626 may include audio/ visual components 634 (e.g., a display such as a plasma display panel (PDP), a light emitting diode (LED) display, a liquid crystal display (LCD), a projector, or a cathode ray tube (CRT)), acoustic components (e.g., speakers), haptic components (e.g., a vibratory motor, resistance mechanisms), other signal generators, and so forth. The input components 628 may include alphanumeric input components (e.g., a keyboard, a touch screen configured to receive alphanumeric input, a photooptical keyboard, or other alphanumeric input components), point based input components (e.g., a mouse, a touchpad, a trackball, a joystick, a motion sensor, or other pointing instrument), tactile input components (e.g., a physical button, a touch screen that provides location and/or force of touches or touch gestures, or other tactile input components), audio/visual input components 636 (e.g., a microphone), and the like.

Communication may be implemented using a wide variety of technologies. The I/O components 602 may include communication components 630 operable to couple the device 600 to a network 622 or devices 624 via coupling 638 and coupling 640, respectively. For example, the communication components 630 may include a network interface component or other suitable device to interface with the network 622. In further examples, communication components 630 may include wired communication components, wireless communication components, cellular communication components, Near Field Communication (NFC) components, Bluetooth^{®} components (e.g., Bluetooth^{®} Low Energy), Wi-Fi^{®} components, and other communication components to provide communication via other modalities. The devices 624 may be another machine or any of a wide variety of peripheral devices (e.g., a peripheral device coupled via a USB).

Moreover, the communication components 630 may detect identifiers or include components operable to detect identifiers. For example, the communication components 630 may include Radio Frequency Identification (RFID) tag reader components, NFC smart tag detection components, optical reader components (e.g., an optical sensor to detect onedimensional bar codes such as Universal Product Code (UPC) bar code, multi-dimensional bar codes such as Quick Response (QR) code, Aztec code, Data Matrix, Dataglyph, MaxiCode, PDF416, Ultra Code, UCC RSS-2D bar code, and other optical codes), or acoustic detection components (e.g., microphones to identify tagged audio signals). In addition, a variety of information may be derived via the communication components 630, such as location via Internet Protocol (IP) geo-location, location via Wi-Fi^{®} signal triangulation, location via detecting an NFC beacon signal that may indicate a particular location, and so forth.

The instructions 610 may be transmitted or received over the network 622 using a transmission medium via a network interface device (e.g., a network interface component included in the communication components 630) and utilizing any one of several well-known transfer protocols (e.g., hypertext transfer protocol (HTTP)). Similarly, the instructions 610 may be transmitted or received using a transmission medium via the coupling 640 (e.g., a peer-to-peer coupling) to devices 624. The term "transmission medium" shall be taken to include any intangible medium that is capable of storing, encoding, or carrying instructions 610 for execution by the device 600, and includes digital or analog communications signals or other intangible medium to facilitate communication of such software.

Although a few embodiments have been described in detail above, other modifications are possible. For example, the logic flows depicted in the figures do not require the particular order shown, or sequential order, to achieve desirable results. Other steps may be provided, or steps may be eliminated, from the described flows, and other components may be added to, or removed from, the described systems. Other embodiments may be within the scope of the following claims.

This summary is intended to provide an overview of subject matter of the present patent application. It is not intended to provide an exclusive or exhaustive explanation of the invention. The detailed description is included to provide further information about the present patent application.

The above detailed description includes references to the accompanying drawings, which form a part of the detailed description. The drawings show, by way of illustration, specific implementations in which the invention can be practiced. These implementations are also referred to generally as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and
"wherein." Also, in the following aspects, the terms "including" and "comprising" are open-ended, that is, a system, device, article, composition, formulation, or process that includes elements in addition to those listed after such a term in an aspect are still deemed to fall within the scope of that aspect. Moreover, in the following aspects, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other implementations can be used, such as by one of ordinary skill in the art upon reviewing the above description. Also, in the above Description, various features may be grouped together to streamline the disclosure. This should not be interpreted as intending that an unclaimed disclosed feature is essential to any aspect. Rather, inventive subject matter may lie in less than all features of a particular disclosed implementation. Thus, the following aspects are hereby incorporated into the Description as examples or implementations, with each aspect standing on its own as a separate implementation, and it is contemplated that such implementations can be combined with each other in various combinations or permutations. The scope of the invention is defined by the appended claims.

## Claims

1. A method, comprising:
receiving a physiologic signal from an accelerometer channel, the signal obtained extra-corporeally at a first time window;
applying a filter on the physiologic signal to obtain a filtered physiologic signal (404);
performing, on the filtered physiologic signal, a beat time estimation within the first time window (406), wherein performing the beat time estimation further comprises:
determining peak samples of the filtered physiologic signal;
determining trough samples of the filtered physiologic signal;
calculating beat times as a function of the peak and trough samples; and
calculating a median of beat times as the beat time estimation;
based on the estimation, calculating a heart rate from the filtered physiologic signal (408); and
at least one of storing or transmitting the heart rate (414).

2. The method of claim 1, further comprising:
continuously receiving the physiologic signal from the accelerometer channel (402);
as the physiologic signal is received:
applying the filter on the physiologic signal to obtain the filtered physiologic signal (404);
performing, on the filtered physiologic signal, the beat time estimation based on a specified time segment (406);
based on the beat time estimation, calculating a heart rate variability, HRV, from the filtered physiologic signal for the specified time segment (410).

3. The method of claim 2, further comprising at least one of storing or transmitting the heart rate variability (414).

4. The method of claim 1, further comprising:
applying a detrending operation on the physiologic signal to generate a detrended physiologic signal;
applying a second filter on the detrended physiologic signal to generate a filtered detrended physiologic signal;
calculating peak-to-peak times of the filtered detrended physiologic signal;
based on the calculated peak-to-peak times, calculating a respiration rate of the physiologic signal (412); and optionally
at least one of storing or transmitting the respiration rate and the filtered detrended physiologic signal (414).

5. The method of claim 4, wherein the second filter is a lowpass filter.

6. The method of claim 1, wherein the filter is a bandpass filter, and the method further comprises applying an equalization operation on the filtered physiologic signal.

7. A signal acquisition system configured to track a physiologic signal, wherein the system is configured to:
receive, a physiologic signal from an accelerometer channel (402), wherein the accelerometer channel is a channel of an accelerometer unit, the signal obtained extra-corporeally at a first time window;
apply, a filter on the physiologic signal to obtain a filtered physiologic signal (404);
perform, on the filtered physiologic signal, a beat time estimation within the first time window (406), wherein performing the beat time estimation further comprises:
determining peak samples of the filtered physiologic signal;
determining trough samples of the filtered physiologic signal;
calculating beat times as a function of the peak and trough samples; and
calculating a median of beat times as the beat time estimation;
based on the estimation, calculate a heart rate from the filtered physiologic signal (408); and
at least one of store or transmit the heart rate (414).

8. The signal acquisition system of claim 7, further comprising:
continuously receiving the physiologic signal from the accelerometer channel (402);
as the physiologic signal is received:
applying the filter on the physiologic signal to obtain the filtered physiologic signal (404);
performing, on the filtered physiologic signal, the beat time estimation based on a specified time segment (406);
based on the beat time estimation, calculating a heart rate variability, HRV, from the filtered physiologic signal for the specified time segment (410).

9. The signal acquisition system of claim 8, further comprising at least one of storing or transmitting the heart rate variability (414).

10. The signal acquisition system of claim 7, further comprising:
applying a detrending operation on the physiologic signal to generate a detrended physiologic signal;
applying a second filter on the detrended physiologic signal to generate a filtered detrended physiologic signal;
calculating peak-to-peak times of the filtered detrended physiologic signal;
based on the calculated peak-to-peak times, calculating a respiration rate of the physiologic signal (412); and
at least one of storing or transmitting the respiration rate and the filtered detrended physiologic signal (414).

11. The signal acquisition system of claim 10, wherein the second filter is a lowpass filter.

12. The signal acquisition system of claim 7, wherein the filter is a bandpass filter, and the system further performs operations comprising applying an equalization operation on the filtered physiologic signal.

13. A non-transitory computer readable storage medium storing one or more programs, the one or more programs comprising instructions which when executed by one or more processors of an electronic device, cause the electronic device to:
receive a physiologic signal from an accelerometer channel, wherein the accelerometer channel is a channel of an accelerometer unit (402), the signal obtained extra-corporeally at a first time window;
apply a filter on the physiologic signal to obtain a filtered physiologic signal (404);
perform, on the filtered physiologic signal, a beat time estimation within the first time window (406), wherein performing the beat time estimation further comprises:
determining peak samples of the filtered physiologic signal;
determining trough samples of the filtered physiologic signal;
calculating beat times as a function of the peak and trough samples; and
calculating a median of beat times as the beat time estimation;
based on the estimation, calculate a heart rate from the filtered physiologic signal (408); and
at least one of storing or transmitting the heart rate (414).

14. The storage medium of claim 13, further comprising:
continuously receiving the physiologic signal from the accelerometer channel (402);
as the physiologic signal is received:
applying the filter on the physiologic signal to obtain the filtered physiologic signal (404);
performing, on the filtered physiologic signal, the beat time estimation based on a specified time segment (406);
based on the beat time estimation, calculating a heart rate variability, HRV, from the filtered physiologic signal for the specified time segment (410).

15. The storage medium of claim 13, further comprising:
applying a detrending operation on the physiologic signal to generate a detrended physiologic signal;
applying a second filter on the detrended physiologic signal to generate a filtered detrended physiologic signal;
calculating peak-to-peak times of the filtered detrended physiologic signal; and
based on the calculated peak-to-peak times, calculating a respiration rate of the physiologic signal (412).

## Patentansprüche

1. Verfahren, das Folgendes umfasst:
Empfangen eines physiologischen Signals von einem Beschleunigungsmesser-Kanal, wobei das Signal in einem ersten Zeitfenster extra-korporal erhalten wird;
Anwenden eines Filters auf das physiologische Signal, um ein gefiltertes physiologisches Signal (404) zu erhalten;
Durchführen einer Schlagzeit-Schätzung innerhalb des ersten Zeitfensters (406) auf dem gefilterten physiologischen Signal, wobei das Durchführen der Schlagzeit-Schätzung ferner Folgendes umfasst:
Bestimmen von Spitzenwerten des gefilterten physiologischen Signals;
Bestimmen von Tiefstwerten des gefilterten physiologischen Signals;
Berechnen von Schlagzeiten als eine Funktion der Spitzen- und Tiefstwerte; und
Berechnen eines Medians der Schlagzeiten als die Schlagzeit-Schätzung;
Berechnen einer Herzfrequenz aus dem gefilterten physiologischen Signal (408)basierend auf der Schätzung; und
Speichern und/oder Übermitteln der Herzfrequenz (414).

2. Verfahren nach Anspruch 1, das ferner Folgendes umfasst:
kontinuierliches Empfangen des physiologischen Signals von dem Beschleunigungsmesser-Kanal (402);
dann, wenn das physiologische Signal empfangen wird:
Anwenden des Filters auf das physiologische Signal, um das gefilterte physiologische Signal (404) zu erhalten;
Durchführen der Schlagzeit-Schätzung auf dem gefilterten physiologischen Signal basierend auf einem spezifischen Zeitsegment (406);
Berechnen einer Herzfrequenz-Variabilität (HRV) aus dem gefilterten physiologischen Signal für das spezifische Zeitsegment (410) basierend auf der Schlagzeit-Schätzung.

3. Verfahren nach Anspruch 2, das ferner der Speichern und/oder Übermitteln der Herzfrequenz-Variabilität (414) umfasst.

4. Verfahren nach Anspruch 1, das ferner Folgendes umfasst:
Anwenden einer Trendkorrektur-Operation auf das physiologische Signal, um ein trendkorrigiertes physiologisches Signal zu erzeugen;
Anwenden eines zweiten Filters auf das trendkorrigierte physiologische Signal, um ein gefiltertes trendkorrigiertes physiologisches Signal zu erzeugen;
Berechnen von Spitze/Spitze-Zeiten des gefilterten trendkorrigierten physiologischen Signals;
Berechnen einer Atemfrequenz des physiologischen Signals (412) basierend auf den berechneten Spitze/Spitze-Zeiten; und wahlweise
Speichern und/oder Übermitteln der Atemfrequenz und des gefilterten trendkorrigierten physiologischen Signals (414).

5. Verfahren nach Anspruch 4, wobei das zweite Filter ein Tiefpassfilter ist.

6. Verfahren nach Anspruch 1, wobei das Filter ein Bandpassfilter ist und das Verfahren ferner das Anwenden einer Ausgleichsoperation auf das gefilterte physiologische Signal umfasst.

7. Signalerfassungssystem, das konfiguriert ist, ein physiologisches Signal zu verfolgen, wobei das System konfiguriert ist:
ein physiologisches Signal von einem Beschleunigungsmesser-Kanal (402) zu empfangen, wobei der Beschleunigungsmesser-Kanal ein Kanal einer Beschleunigungsmesser-Einheit ist, wobei das Signal in einem ersten Zeitfenster extra-korporal erhalten wird;
auf das physiologische Signal ein Filter anzuwenden, um ein gefiltertes physiologisches Signal (404) zu erhalten;
eine Schlagzeit-Schätzung an dem gefilterten physiologischen Signal innerhalb des ersten Zeitfensters (406) durchzuführen, wobei das Durchführen der Schlagzeit-Schätzung ferner Folgendes umfasst:
Bestimmen von Spitzenwerten des gefilterten physiologischen Signals;
Bestimmen von Tiefstwerten des gefilterten physiologischen Signals;
Berechnen von Schlagzeiten als eine Funktion der Spitzen- und Tiefstwerte; und
Berechnen eines Medians der Schlagzeiten als die Schlagzeit-Schätzung;
eine Herzfrequenz aus dem gefilterten physiologischen Signal (408) basierend auf der Schätzung zu berechnen; und
die Herzfrequenz (414) zu speichern und/oder zu übermitteln.

8. Signalerfassungssystem nach Anspruch 7, das ferner Folgendes umfasst:
kontinuierliches Empfangen des physiologischen Signals von dem Beschleunigungsmesser-Kanal (402);
dann, wenn das physiologische Signal empfangen wird:
Anwenden des Filters auf das physiologische Signal, um das gefilterte physiologische Signal (404) zu erhalten;
Durchführen der Schlagzeit-Schätzung auf dem gefilterten physiologischen Signal basierend auf einem spezifischen Zeitsegment (406);
Berechnen einer Herzfrequenz-Variabilität (HRV) aus dem gefilterten physiologischen Signal für das spezifische Zeitsegment (410) basierend auf der Schlagzeit-Schätzung.

9. Signalerfassungssystem nach Anspruch 8, das ferner das Speichern und/oder Übermitteln der Herzfrequenz-Variabilität (414) umfasst.

10. Signalerfassungssystem nach Anspruch 7, das ferner Folgendes umfasst:
Anwenden einer Trendkorrektur-Operation auf das physiologischen Signal, um ein trendkorrigiertes physiologisches Signal zu erzeugen;
Anwenden eines zweiten Filters auf das trendkorrigierte physiologische Signal, um ein gefiltertes trendkorrigiertes physiologisches Signal zu erzeugen;
Berechnen von Spitze/Spitze-Zeiten des gefilterten trendkorrigierten physiologischen Signals;
Berechnen einer Atemfrequenz des physiologischen Signals (412) basierend auf den berechneten Spitze/Spitze-Zeiten; und
Speichern und/oder Übermitteln der Atemfrequenz und des gefilterten trendkorrigierten physiologischen Signals (414).

11. Signalerfassungssystem nach Anspruch 10, wobei das zweite Filter ein Tiefpassfilter ist.

12. Signalerfassungssystem nach Anspruch 7, wobei das Filter ein Bandpassfilter ist und das System ferner Operationen durchführt, die das Anwenden einer Ausgleichsoperation auf das gefilterte physiologische Signal umfasst.

13. Nichtflüchtiges computerlesbares Speichermedium, das ein oder mehrere Programme speichert, wobei das eine oder die mehreren Programme Anweisungen enthalten, die dann, wenn sie durch einen oder mehrere Prozessoren einer elektronischen Vorrichtung ausgeführt werden, die elektronische Vorrichtung veranlassen:
ein physiologisches Signal von einem Beschleunigungsmesser-Kanal (402) zu empfangen, wobei der Beschleunigungsmesser-Kanal ein Kanal einer Beschleunigungsmesser-Einheit ist, wobei das Signal in einem ersten Zeitfenster extra-korporal erhalten wird;
ein Filter auf das physiologische Signal anzuwenden, um ein gefiltertes physiologisches Signal (404) zu erhalten;
an dem gefilterten physiologischen Signal eine Schlagzeit-Schätzung innerhalb des ersten Zeitfensters (406) durchzuführen, wobei das Durchführen der Schlagzeit-Schätzung ferner Folgendes umfasst:
Bestimmen von Spitzenwerten des gefilterten physiologischen Signals;
Bestimmen von Tiefstwerten des gefilterten physiologischen Signals;
Berechnen von Schlagzeiten als eine Funktion der Spitzen- und Tiefstwerte; und
Berechnen eines Medians der Schlagzeiten als die Schlagzeit-Schätzung;
eine Herzfrequenz aus dem gefilterten physiologischen Signal (408) basierend auf der Schätzung zu berechnen; und
die Herzfrequenz (414) zu speichern und/oder zu übermitteln.

14. Speichermedium nach Anspruch 13, das ferner Folgendes umfasst:
kontinuierliches Empfangen des physiologischen Signals von dem Beschleunigungsmesser-Kanal (402);
dann, wenn das physiologische Signal empfangen wird:
Anwenden des Filters auf das physiologische Signal, um das gefilterte physiologische Signal (404) zu erhalten;
Durchführen der Schlagzeit-Schätzung an dem gefilterten physiologischen Signal basierend auf einem spezifischen Zeitsegment (406);
Berechnen einer Herzfrequenz-Variabilität (HRV) aus dem gefilterten physiologischen Signal für das spezifische Zeitsegment (410) basierend auf der Schlagzeit-Schätzung.

15. Speichermedium nach Anspruch 13, das ferner Folgendes umfasst:
Anwenden einer Trendkorrektur-Operation auf das physiologische Signal, um ein trendkorrigiertes physiologisches Signal zu erzeugen;
Anwenden eines zweiten Filters auf das trendkorrigierte physiologische Signal, um ein gefiltertes trendkorrigiertes physiologisches Signal zu erzeugen;
Berechnen von Spitze/Spitze-Zeiten des gefilterten trendkorrigierten physiologischen Signals; und
Berechnen einer Atemfrequenz des physiologischen Signals (412) basierend auf den berechneten Spitze/Spitze-Zeiten.

## Revendications

1. Procédé, comprenant les étapes consistant à :
recevoir un signal physiologique provenant d'un canal d'accéléromètre, le signal étant obtenu de manière extracorporelle au niveau d'une première fenêtre temporelle ;
appliquer un filtre au signal physiologique pour obtenir un signal physiologique filtré (404) ;
effectuer, sur le signal physiologique filtré, une estimation du temps de battement dans la première fenêtre temporelle (406), dans lequel l'exécution de l'estimation de temps de battement comprend en outre les étapes consistant à :
déterminer des échantillons de crête du signal physiologique filtré ;
déterminer des échantillons de creux du signal physiologique filtré ;
calculer des temps de battement en fonction des échantillons de crête et de creux ; et
calculer une médiane des temps de battement en tant qu'estimation de temps de battement ;
sur la base de l'estimation, calculer une fréquence cardiaque à partir du signal physiologique filtré (408) ; et
au moins une des étapes consistant à stocker ou transmettre la fréquence cardiaque (414).

2. Procédé selon la revendication 1, comprenant en outre l'étape consistant à :
recevoir en continu le signal physiologique en provenance du canal d'accéléromètre (402) ;
lorsque le signal physiologique est reçu :
appliquer le filtre au signal physiologique pour obtenir le signal physiologique filtré (404) ;
effectuer, sur le signal physiologique filtré, l'estimation du temps de battement sur la base d'un segment temporel spécifié (406) ;
sur la base de l'estimation du temps de battement, calculer une variabilité de fréquence cardiaque, HRV, à partir du signal physiologique filtré pour le segment de temps spécifié (410).

3. Procédé selon la revendication 2, comprenant en outre au moins une des étapes consistant à stocker ou à transmettre la variabilité de fréquence cardiaque (414).

4. Procédé selon la revendication 1, comprenant en outre les étapes consistant à :
appliquer une opération de redressement sur le signal physiologique pour générer un signal physiologique redressé ;
appliquer un second filtre sur le signal physiologique redressé pour générer un signal physiologique redressé filtré ;
calculer les temps de crête à crête du signal physiologique redressé filtré ;
sur la base des temps de crête à crête calculés, calculer une fréquence de respiration du signal physiologique (412) ; et facultativement
au moins une des étapes consistant à stocker ou transmettre la fréquence de respiration et le signal physiologique redressé filtré (414).

5. Procédé selon la revendication 4, lequel le second filtre est un filtre passe-bas.

6. Procédé selon la revendication 1, dans lequel le filtre est un filtre passe-bande, et le procédé comprend en outre l'application d'une opération d'égalisation sur le signal physiologique filtré.

7. Système d'acquisition de signal configuré pour suivre un signal physiologique, dans lequel le système est configuré pour :
recevoir un signal physiologique provenant d'un canal d'accéléromètre (402), dans lequel le canal d'accéléromètre est un canal d'une unité d'accéléromètre, le signal étant obtenu de manière extracorporelle au niveau d'une première fenêtre temporelle ;
appliquer un filtre au signal physiologique pour obtenir un signal physiologique filtré (404) ;
effectuer, sur le signal physiologique filtré, une estimation du temps de battement dans la première fenêtre temporelle (406), dans lequel l'exécution de l'estimation du temps de battement comprend en outre les étapes consistant à :
déterminer des échantillons de crête du signal physiologique filtré ;
la détermination des échantillons de creux du signal physiologique filtré ;
calculer des temps de battement en fonction des échantillons de crête et de creux ; et
calculer une médiane des temps de battement en tant qu'estimation de temps de battement ;
sur la base de l'estimation, calculer une fréquence cardiaque à partir du signal physiologique filtré (408) ; et
au moins une des étapes consistant à stocker ou transmettre la fréquence cardiaque (414).

8. Système d'acquisition de signaux selon la revendication 7, comprenant en outre l'étape consistant à :
recevoir en continu le signal physiologique en provenance du canal d'accéléromètre (402) ;
lorsque le signal physiologique est reçu :
appliquer le filtre au signal physiologique pour obtenir le signal physiologique filtré (404) ;
effectuer, sur le signal physiologique filtré, l'estimation du temps de battement sur la base d'un segment temporel spécifié (406) ;
sur la base de l'estimation du temps de battement, calculer une variabilité de la fréquence cardiaque, HRV, à partir du signal physiologique filtré pour le segment temporel spécifié (410).

9. Système d'acquisition de signal selon la revendication 8, comprenant en outre au moins une des étapes consistant à stocker ou à transmettre la variabilité de fréquence cardiaque (414).

10. Système d'acquisition de signaux selon la revendication 7, comprenant en outre l'étape consistant à :
appliquer une opération de redressement sur le signal physiologique pour générer un signal physiologique redressé ;
appliquer un second filtre sur le signal physiologique redressé pour générer un signal physiologique redressé filtré ;
calculer les temps de crête à crête du signal physiologique redressé filtré ;
sur la base des temps de crête à crête calculés, calculer une fréquence de respiration du signal physiologique (412) ; et
au moins une des étapes consistant à stocker ou transmettre la fréquence de respiration et le signal physiologique redressé filtré (414).

11. Système d'acquisition de signal selon la revendication 10, dans lequel le second filtre est un filtre passe-bas.

12. Système d'acquisition de signal selon la revendication 7, dans lequel le filtre est un filtre passe-bande, et le système effectue en outre des opérations comprenant l'application d'une opération d'égalisation sur le signal physiologique filtré.

13. Support de stockage non transitoire lisible par ordinateur stockant un ou plusieurs programmes, les un ou plusieurs programmes comprenant des instructions qui, lorsqu'elles sont exécutées par un ou plusieurs processeurs d'un dispositif électronique, amènent le dispositif électronique à :
recevoir un signal physiologique provenant d'un canal d'accéléromètre, dans lequel le canal d'accéléromètre est un canal d'une unité d'accéléromètre (402), le signal étant obtenu de manière extracorporelle au niveau d'une première fenêtre temporelle ;
appliquer un filtre au signal physiologique pour obtenir un signal physiologique filtré (404) ;
effectuer, sur le signal physiologique filtré, une estimation du temps de battement dans la première fenêtre temporelle (406), dans lequel l'exécution de l'estimation du temps de battement comprend en outre les étapes consistant à :
déterminer des échantillons de crête du signal physiologique filtré ;
déterminer des échantillons de creux du signal physiologique filtré ;
calculer des temps de battement en fonction des échantillons de crête et de creux ; et
calculer une médiane des temps de battement en tant qu'estimation de temps de battement ;
sur la base de l'estimation, calculer une fréquence cardiaque à partir du signal physiologique filtré (408) ; et
au moins une des étapes consistant à stocker ou transmettre la fréquence cardiaque (414).

14. Support de stockage selon la revendication 13, comprenant en outre les étapes consistant à :
recevoir en continu le signal physiologique en provenance du canal d'accéléromètre (402) ;
lorsque le signal physiologique est reçu :
appliquer le filtre au signal physiologique pour obtenir le signal physiologique filtré (404) ;
effectuer, sur le signal physiologique filtré, l'estimation du temps de battement sur la base d'un segment temporel spécifié (406) ;
sur la base de l'estimation du temps de battement, calculer une variabilité de la fréquence cardiaque, HRV, à partir du signal physiologique filtré pour le segment temporel spécifié (410).

15. Support de stockage selon la revendication 13, comprenant en outre les étapes consistant à :
appliquer une opération de redressement sur le signal physiologique pour générer un signal physiologique redressé ;
appliquer un second filtre sur le signal physiologique redressé pour générer un signal physiologique redressé filtré ;
calculer les temps de crête à crête du signal physiologique redressé filtré ; et
sur la base des temps crête à crête calculés, calculer une fréquence de respiration du signal physiologique (412).
